(19) Europäisches Patentamt
European Patent Office
Office européen des brevets

(11) Numéro de publication: **0 280 603**
**A1**

(12) **DEMANDE DE BREVET EUROPEEN**

(21) Numéro de dépôt: **88400253.6**

(22) Date de dépôt: **03.02.88**

(51) Int. Cl.[4]: **C 07 D 453/02**

(30) Priorité: **04.02.87 FR 8701355**

(43) Date de publication de la demande:
**31.08.88 Bulletin 88/35**

(84) Etats contractants désignés:
**BE CH DE ES GB IT LI LU NL SE**

(71) Demandeur: **DELALANDE S.A.**
**32, rue Henri Regnault**
**F-92400 Courbevoie (FR)**

(72) Inventeur: **Dorme, Nicole**
**106, rue de Longchamp**
**F-75016 Paris (FR)**

**Renaud, Alain**
**16, place des Arts**
**F-92500 Rueil Malmaison (FR)**

**Langlois, Michel**
**4, place César Franck**
**F-78530 Buc (FR)**

(74) Mandataire: **Kedinger, Jean-Paul et al**
**c/o Cabinet Malemont 42, avenue du Président Wilson**
**F-75116 Paris (FR)**

(54) **Enantiomères de configuration absolue S de dérivés amide de l'amino-3 quinuclidine, leur procédé de préparation et leur application en thérapeutique.**

(57) Enantiomère de configuration absolue S de formule :

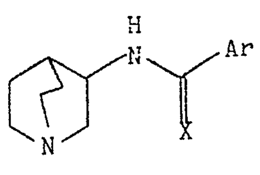

(I)

où :
. X = O ou S ; et
. Ar représente un :
* noyau phényle substitué par un, deux ou trois groupes alkoxy en $C_1$-$C_4$ ;
* noyau phényle substitué en position 2 par un groupe$OCH_3$ et en position 5 par un atome d'halogène ou un groupe alkylcarbonyle inférieur ;
* noyau phényle substitué en position 2 par $OCH_3$, en position 4 par $NH_2$ ou alkylcarbonyle -NH- et en position 5 par un halogène ;
* groupe (fluoro-3 méthoxy-2) phényle ;
* groupe pyrimidynyle-5 substitué en position 2 par $NH_2$ et en position 4 par alkoxy ou phényloxy.

Ces composés sont utiles en tant que médicaments à activité gastrocinétique et antiémétique.

EP 0 280 603 A1

**Description**

Enantiomères de configuration absolue S dérivés amide de l'amino-3 quinuclidine, leur procédé de préparation et leur application en thérapeutique

La présente invention a pour objet les énantiomères de configuration absolue S de dérivés amide de l'amino-3 quinuclidine, leur procédé de préparation et leur application en thérapeutique.

Par EP-A-0099789 on connait déjà des dérivés amide de l'amino-3 quinuclidine, répondant à la formule :

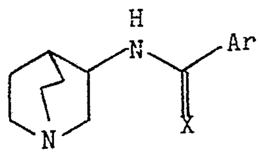

(I)

dans laquelle :

-X représente un atome d'oxygène ou de soufre ; et

-Ar a l'une des significations suivantes :

*) noyau phényle substitué par un, deux ou trois groupes alkoxy en $C_1$-$C_4$ ;

*) noyau phényle de structure :

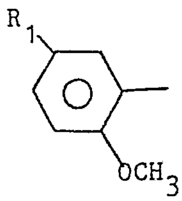

où $R_1$ représente un atome d'halogène ou un groupe alkylcarbonyle dont le groupe alkyle comporte de 1 à 4 atomes de carbone ;

*) groupe phényle de structure :

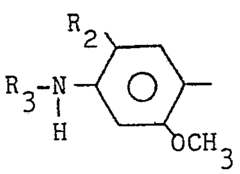

où $R_2$ représente un atome d'halogène et $R_3$ est un atome d'hydrogène ou un groupe alkylcarbonyle dont le reste alkyle comporte 1 à 4 atomes de carbone ;

*) groupe (fluor-3 méthoxy-2) phényle ; ou

*) groupe pyrimidinyle-5 de structure :

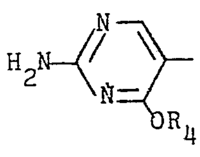

où $R_4$ représente un groupe alkyle en $C_1$-$C_4$ ou le noyau phényle, les sels d'addition d'acide organique ou minéral de ces dérivés, ainsi que les hydrates de ces dérivés et sels.

Les dérivés de formule (I), sels et hydrates ci-dessus n'ont toutefois été décrits, dans EP-A-0099789, que sous la forme de leur mélange racémique. Au cours de l'étude du mélange racémique de ces composés, la Demanderesse a cherché à synthétiser les deux énantiomères correspondant à chaque mélange racémique. Après avoir surmonté le problème posé par cette synthèse, elle a étudié les propriétés pharmacologiques de ces énantiomères et elle a ainsi pu constater que, de façon tout à fait inattendue, les énantiomères de configuration absolue S avaient un comportement pharmacologique très différent aussi bien des mélanges racémique correspondants que de leurs antipodes de configuration absolue R.

Par conséquent, la présente invention a pour objet les énantiomères de configuration absolue S des dérivés

de formule (I), sels et hydrates ci-dessus. Elle a en outre pour objet les procédés de préparation de ces énantiomères ainsi que l'application en thérapeutique de ces énantiomères.

Les énantiomères selon l'invention peuvent être obtenus par dédoublement de mélanges racémique ou par synthèse stéréospécifique.

La synthèse stéréospécifique consiste :

(i) soit à condenser l'amino-3 quinuclidine lévogyre de configuration S respectivement sur les acides ou thioacides de formule :

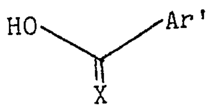

(III)

où X et Ar ont la même signification que dans la formule (I), notamment en présence de dicyclohexylcarbodiimide,

(ii) soit à condenser l'amino-3 quinuclidine lévogyre de configuration S respectivement sur le chlorure des acides ou thioacides de formule :

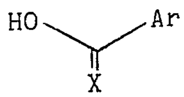

(II)

où X a la même signification que dans la formule (I) et Ar′ prend l'une des significations suivantes :

*) noyau phényle substitué par un, deux ou trois groupes alkoxy en $C_1$-$C_4$ ;

*) noyau phényle de structure :

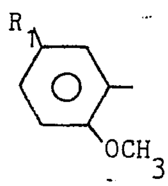

où $R_1$ représente un atome d'halogène ou un groupe alkylcarbonyle dont le groupe alkyle comporte de 1 à 4 atomes de carbone ;

*) groupe phényle de structure :

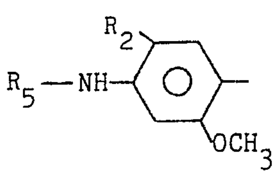

où $R_2$ représente un atome d'halogène et $R_5$ est un groupe alkylcarbonyle dont le reste alkyle comporte 1 à 4 atomes de carbone ;

*) groupe (fluoro-3 méthoxy-2) phényle ; ou

*) groupe pyrimidinyle-5 de structure :

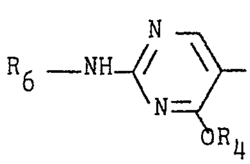

où $R_4$ représente un groupe alkyle en $C_1$-$C_4$ ou le noyau phényle et $R_6$ représente un groupe acétamido, ce qui conduit aux énantiomères de configuration absolue S de formule :

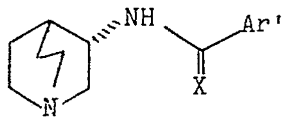

(Ia)

où X et Ar′ ont la même signification que dans la formule (III), puis si besoin est à soumettre les énantiomères de formule (Ia) et de structures particulières :

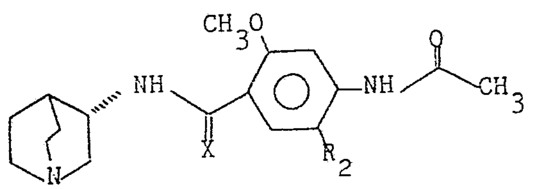

,

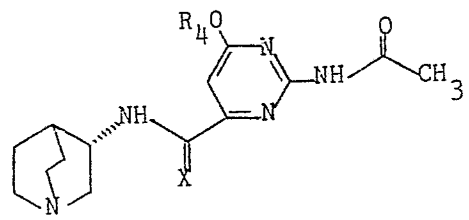

où $R_2$ et $R_4$ ont la même signification que dans la formule (Ia), à une réaction de désacétylation, de préférence par action d'une base forte telle que l'hydroxyde de potassium.

L'amino-3 quinuclidine lévogyre de configuration absolue S est également un composé nouveau puisque seul le mélange racémique de l'amino-3 quinuclidine est connu d'après EP-A-0099789. Elle entre donc également dans le cadre de la présente invention. Elle peut être préparée par deux voies différentes, à savoir :

(i) action de l'acide D-tartrique sur le N-(quinuclidinyl-3) chloro-3 benzamide, dédoublement par cristallisation des sels diastéréoisomères ainsi formés pour obtenir le D-tartrate du N-(quinuclidinyl-3) chloro-3 benzamide lévogyre, action d'une base telle qu'un hydroxyde de métal alcalin sur ce tartrate pour obtenir le S(-) N-(quinuclidinyl-3) chloro-3 benzamide, puis hydrolyse acide de cet amide S(-), ou

(ii) réaction de la quinuclidinone avec la R α-méthylbenzylamine pour obtenir la R N-(α-méthylbenzyl) quinuclidinimine-3, réduction de cette imine par un borohydrure de métal alcalin pour obtenir la R N-(α-méthylbenzyl) S amino-3 quinuclidine, puis hydrogénolyse en milieu acide de cette dernière.

On notera que l'amino-3 quinuclidine dextrogyre de configuration absolue R peut être obtenu par la voie (i) ci-dessus mais en utilisant l'acide L-tartrique au lieu de l'acide D-tartrique ou par la voie (ii) ci-dessus mais en utilisant la S α-méthylbenzylamine au lieu de la R α-méthylbenzylamine.

Les préparation ci-après sont données à titre d'exemple pour illustrer l'invention.

Exemple 1 Préparation de l'amino-3 quinuclidine S(-)

A/ Première voie de synthèse (dédoublement des sels diastéréoisomères):

1ère étape: Préparation du S(-) N-(quinuclidinyl-3) chloro-3 benzamide

Le N-(quinuclidinyl-3) chloro-3 benzamide (52,5 g) en solution dans le méthanol est ajouté à une solution d'acide D-tartrique (29,7 g) dans le méthanol. Le précipité obtenu est recueilli par filtration et traité deux fois par du méthanol au reflux. Le sel ainsi purifié (20 g) est décomposé par une solution aqueuse de soude et le produit extrait au chloroforme. Après séchage et évaporation de la phase organique, la base obtenue est traitée dans l'acétone par une solution d'éthanol chlorhydrique. Le chlorhydrate qui précipite est recueilli par filtration et recristallisé dans l'éthanol. On obtient 9,4 g du chlorhydrate de S(-) N-(quinuclidinyl-3) chloro-3 benzamide optiquement pur.

. Point de fusion : 244° -247° C

$[\alpha]_D^{20}$ = -16,8° (c = 1, $CH_3OH$)

2ème étape: Préparation du S(-) amino-3 quinuclidine dichlorhydrate

Le chlorhydrate obtenu à l'étape précédente (9 g) est traité au reflux pendant 3 heures 30 mn par de l'acide chlorhydrique concentré. Le milieu réactionnel est refroidi, filtré et concentré à sec. Le résidu est traité par l'éthanol absolu et le dichlorhydrate d'amino-3 quinuclidine S(-) qui cristallise est recueilli par filtration :

. Point de fusion : > 260° C

. $[\alpha]_D^{20}$ = - 24,9° (c = 1, $H_2O$).

L'antipode R est obtenu dans les mêmes conditions en utilisant l'acide L-tartrique comme agent de dédoublement :

. Point de fusion > 260° C

. $[\alpha]_D^{20}$ = + 24,8° (c = 1, $H_2O$).

B/ Synthèse asymétrique :

1ère étape: Préparation de la R N-(α-méthylbenzyl) quinuclidinimine-3

La quinuclidinone (80 g) dans 800 ml de toluène est portée au reflux en présence de R α-méthylbenzylamine (77,4 g) pendant 24 h, l'eau formée étant éliminée par un Dean-Stark. Le milieu réactionnel est alors concentré à sec et l'imine formée (130 g) est distillée.

. Rendement : 89 %
. Point d'ébullition = 140 - 150° C (0,05 mm Hg)
. $[\alpha]_D^{20}$ = + 97,2° (c = 1, $CHCl_3$).

2ème étape: Préparation de la R N-(α-méthylbenzyl) S amino-3 quinuclidine dichlorhydrate

L'imine (129,5 g) obtenue à l'étape précédente est dissoute dans du méthanol et du borohydrure de potassium (30,6 g) est ajouté par petites portions entre 10 et 20° C. Après une heure le milieu est évaporé à sec sous pression réduite. Le résidu est dissous dans un mélange d'acétone et d'alcool isopropylique (2/1). L'amine attendue est précipitée sous forme de dichlorhydrate par addition d'une solution d'éthanol chlorhydrique. Le produit est recristallisé deux fois dans un mélange éthanol/méthanol (1/1) pour obtenir le dichlorhydrate de la R N-(α-méthylbenzyl) S amino-3 quinuclidine optiquement pure (81 g).

. Rendement : 47 %
. Point de fusion > 260° C
. $[\alpha]_D^{20}$ = - 2° (c = 2, $H_2O$).

3ème étape: Préparation du dichlorhydrate de la S(-) amino-3 quinuclidine

Le produit obtenu à l'étape précédente (64,4 g) est dissous dans de l'éthanol avec 2 équivalents d'une solution d'acide chlorhydrique 1N et du palladium sur charbon, 50 % $H_2O$ (12,8 g). Le milieu réactionnel est agité 18 h sous atmosphère d'hydrogène, filtré puis évaporé à sec sous pression réduite. Le dichlorhydrate de la S(-) amino-3 quinuclidine est cristallisé dans un mélange éthanol-éther 1/1.

. $[\alpha]_D^{20}$ = - 24,2° (c = 1, $H_2O$).

L'antipode R(+) est obtenu par la même suite de réactions en partant de la S α-méthylbenzylamine.

Exemple 2: Synthèse stéréospécifique du chlorhydrate de la N-(quinuclidinyl-3) amino-4 chloro-5 méthoxy-2 benzamide S(-) [code MD 200054] et R (+) [code MD 200051]

1ère variante:

Le dichlorhydrate de S(-) amino-3 quinuclidine (40 g ; 0,2 mole) est dissous dans 80 ml d'une solution aqueuse de soude 2,5 N. A cette solution refroidie par un bain de glace, est ajouté de l'acide amino-4 chloro-5 méthoxy-2 benzoïque (44,5 g) en solution dans 300 ml de pyridine. De la dicyclohexylcarbodiimide (85 g) est ajoutée en deux fois. Le mélange est agité vigoureusement 18 h à température ambiante. Le milieu est alors dilué par 150 ml d'eau. L'insoluble est éliminé par filtration et lavé à l'eau. La phase aqueuse est amenée à pH 10 par une solution de soude 10 N et extraite par du chloroforme. Après séchage (sur $Na_2SO_4$) et évaporation de la phase organique, le résidu est cristallisé dans l'éther isopropylique.

Le solide obtenu (56 g) est dissous dans 280 ml d'alcool isopropylique et la solution acidifiée par HCl 5N. Le chlorhydrate qui précipite est receuilli par filtration et recristallisé dans l'éthanol à 99 %. Le produit recherché (MD 200054) est obtenu avec un rendement de 60 % :

. Point de fusion : 233-235° C
. $[\alpha]_D^{20}$ = - 3,9° (c = 1, $H_2O$).

L'antipode dextrogyre (MD 200051) est obtenu dans les mêmes conditions à partir du dichlorhydrate de la R(+) amino-3 quinuclidine :

. Point de fusion : 232-234° C
. $[\alpha]_D^{20}$ = + 3,8° (c = 1, $H_2O$).

2ème variante :

L'amino-3 quinuclidine S(-) (1,9 g) est dissoute dans 33,5 ml d'une solution aqueuse de soude 1N. A cette solution est ajouté goutte à goutte le chlorure de l'acide acétamido-4 chloro-5 méthoxy-2 benzoïque (3,75 g) en solution dans 70 ml de dioxane. Après 15 minutes d'agitation, le milieu est acidifié, lavé au chloroforme, alcalinisé avec une solution aqueuse de soude concentrée et le produit extrait au chloroforme. La phase organique est séchée (sur $Na_2SO_4$) puis évaporée. Le résidu huileux est dissous dans l'éthanol et de l'éthanol chlorhydrique est ajouté jusqu'à pH acide. Le chlorhydrate de la N-(quinuclidinyl-3) acétamido-4 chloro-5 méthoxy-2 benzamide ainsi formé précipite (rendement quantitatif) et ce dernier est receuilli par filtration.

Le produit est ensuite désacétylé en le traitant au reflux pendant 30 minutes dans une solution d'hydroxyde de potassium à 5 % dans l'éthanol.

Le milieu réactionnel est ensuite dissous dans l'eau et extrait au chloroforme. Après séchage et évaporation de la phase organique, le chlorhydrate MD 200054 est préparé et isolé comme précédemment.

$[\alpha]_D^{20}$ = - 3,7° (c = 1, $H_2O$).

Les autres énantiomères selon l'invention sont obtenus par des modes opératoires analogues à ceux de l'exemple 2, mais à partir des réactifs de départ appropriés.

Les composés selon l'invention ont été étudiés chez l'animal de laboratoire et ont montré une activité sur le système digestif et notamment une activité gastrocinétique et antiémétique.

A titre d'exemple, on étudie ci-après l'activité antiémétique par voie orale du chlorhydrate du N-(quinuclidinyl-3) amino-4 chloro-5 méthoxy-2 benzamide, sous la forme du mélange racémique (code MD 780209), de l'énantiomère lévogyre de configuration S (code MD 200054) et de l'énantiomère dextrogyre de configuration R (code MD 200051), vis-à-vis des vomissements induits par le cisplatine chez le chien.

1°/ Méthode:

La méthode est dérivée de celle décrite par J.A. GYLYS et coll. (Res. Commun. Chem. Pathol. Pharmacol. 1979, 23, n° 1, p. 61-68). Les vomissements sont induits par injection intraveineuse de cisplatine (DCI) à la dose de 3 mg/kg chez des chiens tout-venants, mâle ou femelle, de poids compris entre 10 et 16 kg, à la diète hydrique depuis 24 heures.

Les composés à tester sont administrés par voie orale 60 minutes après l'injection de cisplatine.

Les animaux sont alimentés 1 h 30 après le traitement. La durée d'observation est de 6 heures après l'injection de cisplatine.

2°/ Résultats (4 chiens par traitement) :

| TRAITEMENT | DOSE µg/kg | VOMISSEMENTS | | |
|---|---|---|---|---|
| | | NOMBRE MOYEN | % DE DIMINUTION EN NOMBRE | % DE DIMINUTION EN TOUT OU RIEN |
| TEMOIN (eau distillée) | 0 | 10,8 | - | - |
| MD 780209 (racémique) | 15 | 2 | 81,4 | 25 |
| MD 200054 (-) | 7,5 | 0,5 | 95,3 | 75 |
| MD 200051 (+) | 30 | 7 | 34,9 | 0 |

Les résultats rassemblés dans le tableau ci-dessus font clairement ressortir que l'énantiomère lévogyre de configuration S (code MD 200054), est de manière surprenante beaucoup plus actif pour inhiber les vomissements induits par des drogues anticancéreuses telles que le cisplatine, que son antipode (code MD 200051) et que le racémique correspondant. En outre, l'énantiomère lévogyre présente moins d'effets secondaires que l'énantiomère dextrogyre.

Les composés selon l'invention ne présentant pas de toxicité significative aux doses actives indiquées dans le tableau ci-dessus, ils trouvent leur application en tant que médicaments dans le domaine du système digestif, notamment comme agents antiémétiques.

La présente invention s'étend enfin aux comositions pharmaceutiques renfermant, à titre de principe actif, au moins un composé choisi parmi les énantiomères de configuration absolue S des dérivés de formule (I), leurs sels d'addition d'acide organique ou minéral pharmaceutiquement acceptables et les hydrates de ces énantiomères et sels, en association avec un véhicule approprié. Ces compositions pourront être administrées par voie orale, sous forme de gélules, comprimés, dragées et similaires, à des doses pouvant atteindre 500 mg de principe actif par jour (en une ou plusieurs prises) ou par voie parentérale sous forme de solutés injectables à des doses pouvant atteindre 200 mg de principe actif par jour (en une ou plusieurs prises journalières).

**Revendications**

1. Enantiomère de configuration absolue S du dérivé amide de l'amino-3 quinuclidine, répondant à la formule :

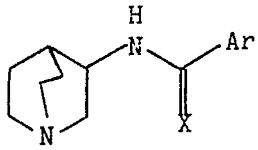

(I)

dans laquelle :
dans laquelle :
-X représente un atome d'oxygène ou de soufre ; et
-Ar a l'une des significations suivantes :
*) noyau phényle substitué par un, deux ou trois groupes alkoxy en $C_1$-$C_4$ ;
*) noyau phényle de structure :

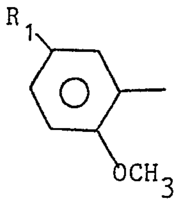

où $R_1$ représente un atome d'halogène ou un groupe alkylcarbonyle dont le groupe alkyle comporte de 1 à 4 atomes de carbone ;
*) groupe phényle de structure :

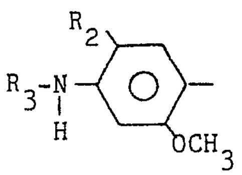

où $R_2$ représente un atome d'halogène et $R_3$ est un atome d'hydrogène ou un groupe alkylcarbonyle dont le reste alkyle comporte 1 à 4 atomes de carbone ;
*) groupe (fluor-3 méthoxy-2) phényle ; ou
*) groupe pyrimidinyle-5 de structure :

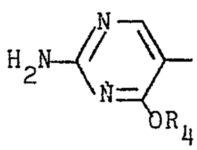

où $R_4$ représente un groupe alkyle en $C_1$-$C_4$ ou le noyau phényle, les sels d'addition d'acide organique ou minéral de ces dérivés, ainsi que l'hydrate de ces dérivé et sels.

2. Enantiomère lévogyre de configuration absolue S du N-(quinuclidinyl-3) amino-4 chloro-5 méthoxy-2 benzamide, de ses sels d'addition d'acide organique ou minéral et de l'hydrate de ce benzamide et de ces sels.

3. Composition pharmaceutique, notamment à activité antiémétique, comprenant au moins un énantiomère selon la revendication 1 ou 2 en association avec un support pharmaceutiquement acceptable.

4. Utilisation de l'énantiomère selon la revendication 1 ou 2 pour la fabrication d'un médicament à activité antiémétique.

5. Procédé de préparation de l'énantiomère selon la revendication 1 ou 2, caractérisé en ce qu'il comprend la condensation de l'amino-3 quinuclidine lévogyre de configuration S sur l'acide ou thioacide de formule :

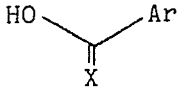

(II)

où X et Ar ont la même signification que dans la formule (I), en présence de dicyclohexylcarbodiimide.

6. Procédé de préparation de l'énantiomère selon la revendication 1 ou 2, caractérisé en ce qu'il comprend les étapes consistant (i) à condenser l'amino-3 quinuclidine lévogyre de configuration S sur le chlorure de l'acide ou thioacide de formule :

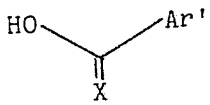

(III)

où X a la même signification que dans la formule (I) et Ar′ prend l'une des significations suivantes :

*) noyau phényle substitué par un, deux ou trois groupes alkoxy en $C_1$-$C_4$ ;

*) noyau phényle de structure :

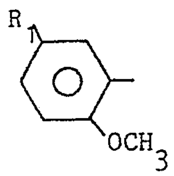

où $R_1$ représente un atome d'halogène ou un groupe alkylcarbonyle dont le groupe alkyle comporte de 1 à 4 atomes de carbone ;

*) groupe phényle de structure :

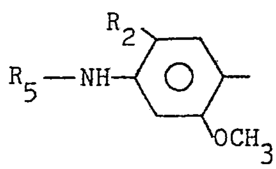

pù $R_2$ représente un atome d'halogene et $R_5$ est un groupe alkylcarbonyle dont le reste alkyle comporte 1 à 4 atomes de carbone ;

*) groupe (fluoro-3 méthoxy-2) phényle ; ou

*) groupe pyrimidinyle-5 de structure :

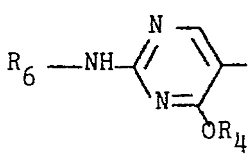

où $R_4$ représente un groupe alkyle en $C_1$-$C_4$ ou le noyau phényle et $R_6$ représente un groupe acétamido, ce qui conduit à l'énantiomère de configuration absolue S de formule :

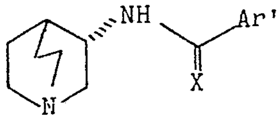

(Ia)

où X et Ar′ ont la même signification que dans la formule (III), puis (ii) si besoin est à soumettre l'énantiomère de formule (Ia) et de structure particulière :

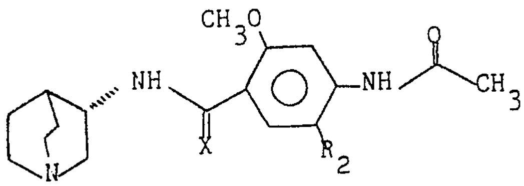

ou

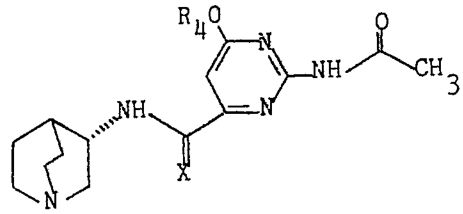

où $R_2$ et $R_4$ ont la même signification que dans la formule (Ia), à une réaction de désacétylation.

7. Amino-3 quinuclidine lévogyre de configuration absolue S.

8. Procédé de préparation de l'amino-3 quinuclidine lévogyre de configuration absolue S, caractérisé en ce qu'il comprend les étapes consistant :

(i) à faire réagir l'acide D-tartrique sur le N-(quinuclidinyl-3) chloro-3 benzamide,

(ii) à dédoubler par cristallisation les sels diastéréoisomères ainsi formés pour obtenir le D-tartrate du N-(quinuclidinyl-3) chloro-3 benzamide lévogyre,

(iii) à faire agir une base sur ce tartrate pour obtenir le S(-) N-(quinuclidinyl-3) chloro-3 benzamide, puis

(iv) à soumettre cet amide S(-) à une hydrolyse acide.

9. Procédé de préparation de l'amino-3 quinuclidine lévogyre de configuration absolue S, caractérisé en ce qu'il comprend les étapes consistant :

(i) à faire réagir la quinuclidinone avec la R α-méthylbenzylamine pour obtenir la R N-(α-méthylbenzyl) quinuclidinimine-3,

(ii) à réduire cette imine par un borohydrure de métal alcalin pour obtenir la R N-(α-méthylbenzyl) S amino-3 quinuclidine, puis

(iii) à soumettre cette dernière à une hydrogénolyse en milieu acide.

Revendications pour l'Etat contractant suivant: ES

1. Procédé de préparation de l'énantiomère de configuration absolue S du dérivé amide de l'amino-3 quinuclidine, répondant à la formule :

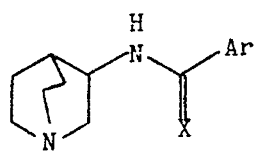

(I)

dans laquelle :

-X représente un atome d'oxygène ou de soufre ; et

-Ar a l'une des significations suivantes :

*) noyau phényle substitué par un, deux ou trois groupes alkoxy en $C_1$-$C_4$ ;

*) noyau phényle de structure :

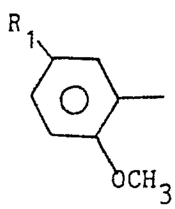

où $R_1$ représente un atome d'halogène ou un groupe alkylcarbonyle dont le groupe alkyle comporte de 1 à 4 atomes de carbone ;

*) groupe phényle de structure :

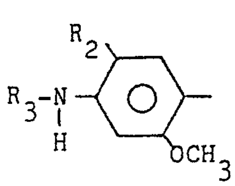

où $R_2$ représente un atome d'halogène et $R_3$ est un atome d'hydrogène ou un groupe alkylcarbonyle dont

le reste alkyle comporte 1 à 4 atomes de carbone ;

*) groupe (fluoro-3 méthoxy-2) phényle ; ou

*) groupe pyrimidinyle-5 de structure :

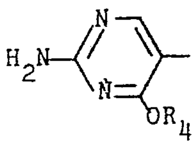

où $R_4$ représente un groupe alkyle en $C_1$-$C_4$ ou le noyau phényle et des sels d'addition d'acide organique ou minéral de ce dérivé, caractérisé en ce qu'il comprend :

(i) la condensation de l'amino-3 quinuclidine lévogyre de configuration S sur l'acide ou thioacide de formule :

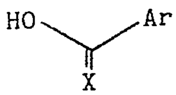 (II)

où X et Ar ont la même signification que dans la formule (I), en présence de dicyclohexylcarbodiimide, ou

(ii) la condensation de l'amino-3 quinuclidine lévogyre de configuration S sur le chlorure de l'acide ou thioacide de formule :

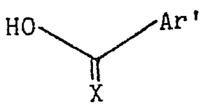 (III)

où X a la même signification que dans la formule (I) et Ar′ prend l'une des significations suivantes :

*) noyau phényle substitué par un, deux ou trois groupes alkoxy en $C_1$-$C_4$ ;

*) noyau phényle de structure:

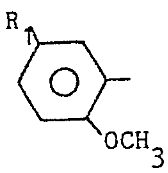

où $R_1$ représente un atome d'halogène ou un groupe alkylcarbonyle dont le groupe alkyle comporte de 1 à 4 atomes de carbone ;

*) groupe phényle de structure :

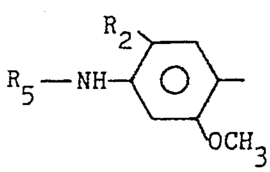

où $R_2$ représente un atome d'halogène et $R_5$ est un groupe alkylcarbonyle dont le reste alkyle comporte 1 à 4 atomes de carbone ;

*) groupe (fluoro-3 méthoxy-2) phényle ; ou

*) groupe pyrimidinyle-5 de structure :

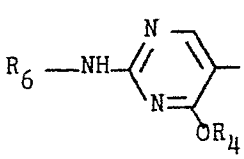

où $R_4$ représente un groupe alkyle en $C_1$-$C_4$ ou le noyau phényle et $R_6$ représente un groupe

acétamido,
ce qui conduit à l'énantiomère de configuration absolue S de formule :

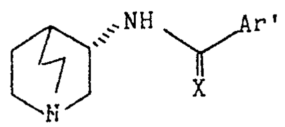

(Ia)

où X et Ar' ont la même signification que dans la formule (III), puis si besoin est la désacétylation de l'énantiomère de formule (Ia) et de structure particulière :

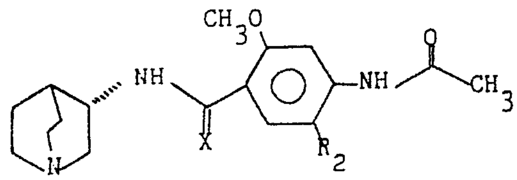

ou

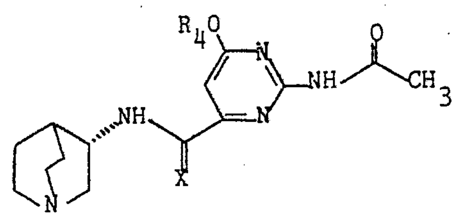

où $R_2$ et $R_4$ ont la même signification que dans la formule (Ia), et

(iii) la transformation de l'énantiomère (I) obtenu en ses sels d'addition d'acide organique ou minéral.

2. Procédé selon la revendication 1, pour la préparation de l'énantiomère lévogyre de configuration absolue S du N-(quinuclidinyl-3) amino-4 chloro-5 méthoxy-2 benzamide et de ses sels d'addition d'acide organique ou minéral.

3. Procédé de préparation d'une composition pharmaceutique ayant notamment une activité antiémétique, caractérisé en ce qu'il consiste à mélanger un énantiomère de configuration absolue S du dérivé amide de l'amino-3 quinuclidine, de formule :

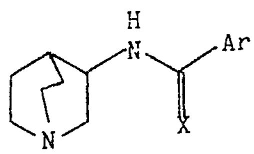

(I)

dans laquelle :
-X représente un atome d'oxygène ou de soufre ; et
-Ar a l'une des significations suivantes :

*) noyau phényle substitué par un, deux ou trois groupes alkoxy en $C_1$-$C_4$ ;

*) noyau phényle de structure :

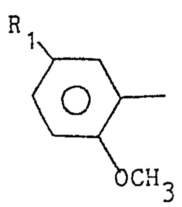

où $R_1$ représente un atome d'halogène ou un groupe alkylcarbonyle dont le groupe alkyle comporte de 1 à 4 atomes de carbone ;

*) groupe phényle de structure :

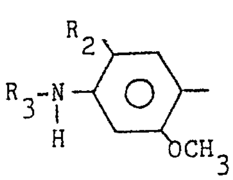

où $R_2$ représente un atome d'halogène et $R_3$ est un atome d'hydrogne ou un groupe alkylcarbonyle dont le reste alkyle comporte 1 à 4 atomes de carbone ;

*) groupe (fluoro-3 méthoxy-2) phényle ; ou

*) groupe pyrimidinyle-5 de structure :

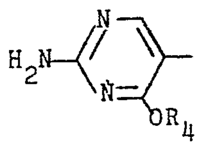

où $R_4$ représente un groupe alkyle en $C_1$-$C_4$ ou le noyau phényle et/ou l'un au moins des sels d'addition d'acide organique ou minéral de ce dérivé, avec un support pharmaceutiquement acceptable et approprié pour ces dérivé et sels. 4. Utilisation de l'énantiomère de configuration absolue S du dérivé de l'amino-3 quinuclidine, de formule :

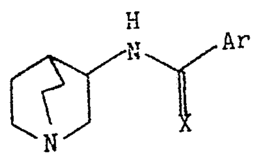

(I)

dans laquelle :
-X représente un atome d'oxygène ou de soufre ; et
-Ar a l'une des significations suivantes :
*) noyau phényle substitué par un, deux ou trois groupes alkoxy en $C_1$-$C_4$ ;
*) noyau phényle de structure :

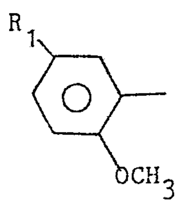

où $R_1$ représente un atome d'halogène ou un groupe alkylcarbonyle dont le groupe alkyle comporte de 1 à 4 atomes de carbone ;
*) groupe phényle de structure :

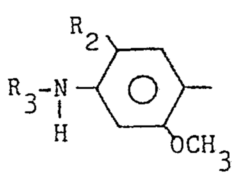

où $R_2$ représente un atome d'halogène et $R_3$ est un atome d'hydrogène ou un groupe alkylcarbonyle dont le reste alkyle comporte 1 à 4 atomes de carbone ;
*) groupe (fluoro-3 méthoxy-2) phényle ; ou
*) groupe pyrimidinyle-5 de structure :

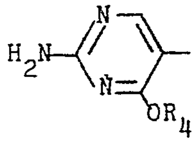

où $R_4$ représente un groupe alkyle en $C_1$-$C_4$ ou le noyau phényle ou d'un sel d'addition d'acide organique ou minéral de ce dérivé, pour la fabrication d'un médicament à activité antiémétique.

5. Procédé de préparation de l'amino-3 quinuclidine lévogyre de configuration absolue S, caractérisé en ce qu'il est choisi entre :
a) le procédé consistant :
(i) à faire réagir l'acide D-tartrique sur le N-(quinuclidinyl-3) chloro-3 benzamide,
(ii) à dédoubler par cristallisation les sels diastéréoisomères ainsi formés pour obtenir le D-tartrate du N-(quinuclidinyl-3) chloro-3 benzamide lévogyre,
(iii) à faire agir une base sur ce tartrate pour obtenir le S(-) N-(quinuclidinyl-3) chloro-3 benzamide,

puis

(iv) à soumettre cet amide S(-) à une hydrolyse acide, et b) le procédé consistant :

(i) à faire réagir la quinuclidinone avec la R α-méthylbenzylamine pour obtenir la R N-(α-méthylbenzyl) quinuclidinimine-3,

(ii) à réduire cette imine par un borohydrure de métal alcalin pour obtenir la R N-(α-méthylbenzyl) S amino-3 quinuclidine, puis

(iii) soumettre cette dernière à une hydrogénolyse en milieu acide.

Office européen des brevets

# RAPPORT DE RECHERCHE EUROPEENNE

Numero de la demande

EP 88 40 0253

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,X | EP-A-0 099 789 (DELALANDE)<br>* Revendications 1,6 *<br>----- | 1,3 | C 07 D 453/02 |
| | | | **DOMAINES TECHNIQUES RECHERCHES (Int. Cl.4)** |
| | | | C 07 D 453/00<br>A 61 K 31/00 |

Le présent rapport a été établi pour toutes les revendications

| Lieu de la recherche | Date d'achèvement de la recherche | Examinateur |
|---|---|---|
| LA HAYE | 16-05-1988 | ALFARO I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)